Europäisches Patentamt

European Patent Office

Office européen des brevets

(11) Publication number : **0 605 983 A2**

(12) # EUROPEAN PATENT APPLICATION

(21) Application number : 93310257.6

(51) Int. Cl.⁵ : **C07K 7/40,** A61K 37/26

(22) Date of filing : 17.12.93

(30) Priority : **18.12.92 US 995659**

(43) Date of publication of application :
**13.07.94 Bulletin 94/28**

(84) Designated Contracting States :
**AT BE CH DE DK ES FR GB GR IE IT LI LU NL
PT SE**

(71) Applicant : **ELI LILLY AND COMPANY
Lilly Corporate Center
Indianapolis Indiana 46285 (US)**

(72) Inventor : **Chance, Ronald Eugene
19303 Flippin Road
Westfield, Indiana 46074 (US)**
Inventor : **Fan, Li
847 E. 57th Street,
Apartment No.3
Chicago, Illinois 60637 (US)**

(74) Representative : **Hudson, Christopher Mark et
al
Erl Wood Manor
Windlesham Surrey GU20 6PH (GB)**

(54) Insulin analogs.

(57)   Analogs of human insulin containing an Ala, Gly, Val, Leu, Ile and Pro residue at position 26 of the B chain, and optionally, having modifications at other positions, display modified physico-chemical and pharmacokinetic properties. These analogs are useful in the treatment of hyperglycemia because they are stable and display a low tendency to self-aggregate.

EP 0 605 983 A2

Jouve, 18, rue Saint-Denis, 75001 PARIS

The present invention is in the field of human medicine, particularly in the treatment of diabetes. Most specifically, the invention relates to analogs of the human insulin molecule, methods of using these analogs and pharmaceutical compositions comprising these insulin analogs.

Diabetes mellitus is a metabolic disorder characterized by the failure of body tissues to oxidize carbohydrates at the normal rate. A deficiency of insulin is the most important factor in the diabetic disease state. During the last 70 years people suffering from diabetes have been greatly aided by receiving controlled amounts of insulin. Until the early 1980's, the insulin used by diabetics was isolated from animal pancreases, generally bovine and porcine. With the introduction of recombinant DNA technology it has become possible to produce large amounts of natural human insulin as well as naturally and non-naturally occurring analogs of human insulin. These insulin analogs display different physical and chemical properties when compared to natural human insulin.

Natural human insulin contains a tyrosine residue at position 26 of the B-chain. Replacement of this tyrosine residue with an alanine residue creates an analog, Ala (B26) human insulin, which demonstrates a lower tendency to self-associate in solution than does natural human insulin. This Ala (B26) human insulin analog also is more stable and has a faster rate of action than doesnormal human insulin. The residue at B26 may also be replaced by glycine, valine, leucine, isoleucine and proline.

The present invention relates to an analog of human insulin modified by changing amino acid 26 of the native human insulin B chain from tyrosine to alanine, glycine, valine, leucine, isoleucine or proline. This molecule may also be modified at position 21 of the native human insulin A-chain and at both positions 1 and 3 of the native human insulin B-chain. Said insulin analogs are more stable and less prone to dimerization or self-association to higher molecular weight forms and thereby possess a comparatively more rapid onset of activity while retaining the biological activity of native human insulin.

Also disclosed and claimed is a method of treating hyperglycemia by administering to a patient in need thereof an effective amount of the claimed analogs. Further, pharmaceutical compositions containing an effective amount of an insulin analog of the invention in combination with one or more pharmaceutically acceptable excipients are disclosed and claimed.

For purposes of the present invention, as disclosed and claimed herein, the following terms and abbreviations are as defined below.

Ala(B26) HI - a human insulin analog wherein the tyrosine residue at position 26 of the B-chain of native human insulin has been changed to an alanine residue.

BHI - biosynthetic human insulin.

Cross-link - the formation of disulfide bonds between cysteine residues. A properly cross-linked native human insulin or insulin analog contains three disulfide bridges. The first disulfide bridge is found between the cysteine residues at positions 6 and 11 of the A-chain. The second disulfide bridge links the cysteine at position 7 of the A-chain to the cysteine at position 7 of the B-chain. The third disulfide bridge links the cysteine at position 20 of the A-chain to the cysteine at position 19 of the B-chain.

Gly(B26) HI - a human insulin analog wherein the tyrosine residue at position 26 of the B-chain of native human insulin has been changed to a glycine residue.

Ile(B26) HI - a human insulin analog wherein the tyrosine residue at position 26 of the B-chain of native human insulin has been changed to an isoleucine residue.

Leu(B26) HI - a human insulin analog wherein the tyrosine residue at position 26 of the B-chain of native human insulin has been changed to a leucine residue.

Pro(B26) HI - a human insulin analog wherein the tyrosine residue at position 26 of the B-chain of native human insulin has been changed to a proline residue.

SEQ ID NO:1 - the first sequence set forth in the sequence listing which is an analog of the human insulin A-chain with the sequence

```
Gly Ile Val Glu Gln Cys Cys Thr Ser Ile Cys Ser Leu Tyr Gln
 1               5                   10                      15
Leu Glu Asn Tyr Cys Xaa
                20
```

wherein Xaa is Asn, Asp, Glu, Gln, Ala, Gly or Ser.

SEQ ID NO:2 - the second sequence set forth in the sequence listing which is an analog of the human insulin B-chain with the sequence

```
Xaa Val Xaa Gln His Leu Cys Gly Ser His Leu Val Glu Ala Leu
 1               5                  10                    15


Tyr Leu Val Cys Gly Glu Arg Gly Phe Phe Xaa Thr Pro Lys Thr
                20                   25
```

wherein Xaa at position 1 is Phe or Asp, Xaa at position 3 is Asn or Asp and Xaa at position 26 is Ala, Gly, Val, Leu, Ile or Pro.

Val(B26) HI - a human insulin analog wherein the tyrosine residue at position 26 of the B-chain of native human insulin has been changed to a valine residue.

All amino acid abbreviations used in this disclosure are those accepted by the United States Patent and Trademark Office as set forth in 37 C.F.R. §1.822(b)(2) (1990).

The present invention relates to insulin analogs of the formula

```
                          SEQ ID NO:1


(Gly Ile Val Glu Glu Cys Cys Thr Ser Ile Cys Ser Leu Tyr Gln Leu Glu
  1               5                  10                    15


Asn Tyr Cys Xaa)
       20
```

```
                   properly cross-linked to
                          SEQ ID NO:2


(Xaa Val Xaa Gln His Leu Cys Gly Ser His Leu Val Glu Ala Leu Tyr Leu
  1               5                  10                    15


Val Cys Gly Glu Arg Gly Phe Phe Xaa Thr Pro Lys Thr)
        20                   25
```

or a pharmaceutically acceptable salt thereof, wherein Xaa at position 21 of SEQ ID NO:1 (insulin A-chain) is Asn, Asp, Glu, Gln, Ala, Gly or Ser; Xaa at position 1 of SEQ ID NO:2 (insulin B-chain) is Phe or Asp; Xaa at position 3 of SEQ ID NO:2 is Asn or Asp and Xaa at position 26 of SEQ ID NO:2 is Ala, Gly, Val, Leu, Ile or Pro. Preferably, Xaa at position 21 of SEQ ID NO:1 is Gly, Ala, Ser or Asn. The preferred amino acid residues at position 26 of the B chain (SEQ ID NO:2) are Ala and Gly, while the most preferred residue at this position is Ala. Asparagine is the most particularly preferred amino acid at position 21. One analog of the present invention can be created by removing the amino acid residue normally found at position 1 of the insulin B chain (SEQ ID NO:2). The preferred amino acid at position 1 of SEQ ID NO:2 is Phe and the most preferred residue at position 3 of SEQ ID NO:2 is Asn. A particularly preferred insulin analog of the present invention is one wherein Xaa at position 21 of SEQ ID NO:1 is Asn, Xaa at position 1 of SEQ ID NO:2 is Phe, Xaa at position 3 of SEQ ID NO:2 is Asn and Xaa at position 26 of SEQ ID NO:2 is Ala. In standard biochemical terms known to the skilled artisan said particularly preferred analog is Ala (B26) Human Insulin.

The insulin analogs of the present invention have a reduced propensity to dimerize or otherwise self-associate to higher molecular weight forms, either in a solution which contains zinc or a solution which is zinc-free. In that the analogs are generally monomeric in solution, a rapid onset of activity is achieved upon administration.

As mentioned hereinabove, the invention includes pharmaceutically acceptable salts of the insulin analogs. Preferred such salts are those of zinc, sodium, potassium, magnesium, calcium, or combinations of these salts.

The insulin analogs of this invention can be prepared by any of a variety of recognized peptide synthesis techniques including classical (solution) methods, solid-phase methods, semisynthetic methods and the more recently available recombinant DNA methods.

In the solid-phase technique, the amino acid sequence is constructed sequentially from an initial, insoluble, resin-supported C-terminal amino acid. Techniques for the solid phase method are described by J. Stewart et al., Solid-Phase Peptide Synthesis, Freeman and Co., San Francisco, 1969.

In general, in the solid-phase method, the amino acid corresponding to the C-terminal amino acid residue of the desired peptide is anchored to an insoluble resin support, and the peptide chain then is formed beginning at the resin-supported C-terminal amino acid. Individual amino acids are introduced sequentially until the desired amino acid sequence is obtained. Alternatively, small peptide fragments can be prepared and introduced into the peptide chain in the desired order. The peptide chain remains attached to the resin throughout synthesis, and, upon completion of the chain the peptide is cleaved from the resin.

The peptide chain is attached to the polystyrene resin by means of an ester linkage formed between the carboxyl group of the C-terminal moiety and a specific methylene group present on the resin matrix as a site for such attachment.

The amino acids are coupled using techniques well known in the art for the formation of peptide bonds. One method involves converting the amino acid to a derivative that will render the carboxyl group more susceptible to reaction with the free N-terminal amino group of the peptide fragment. For example, the amino acid can be converted to a mixed anhydride by reaction of a protected amino acid with ethyl chloroformate, phenyl chloroformate, sec-butyl chloroformate, or isobutyl chloroformate. Alternatively, the amino acid can be converted to an active ester such as a 2,4,5-trichlorophenyl ester, a pentachlorophenyl ester, a p-nitrophenyl ester, a N-hydroxysuccinimide ester, or an ester formed from 1-hydroxybenzotriazole.

Another coupling method involves use of a suitable coupling agent, such as N,N'-dicyclohexylcarbodiimide (DCC) or N,N'-diisopropylcarbodiimide (DIC). Other appropriate coupling agents will be apparent to those skilled in the art. See Schroder and Lubke, The Peptides, Academic Press, 1965, Chapter III which is incorporated herein by reference.

It should be recognized that the a-amino group of each amino acid employed in the peptide synthesis must be protected during the coupling reaction to prevent side reactions involving the reactive $\alpha$-amino function. It should also be recognized that certain amino acids contain reactive side-chain functional groups (e.g., sulfhydryl, $\epsilon$-amino, $\beta$-and $\gamma$-carboxyl, imidazole, guanido and hydroxyl), and that such functional groups must also be protected both during the initial and subsequent coupling steps. Suitable protecting groups are known in the art. See for example, Protective Groups In Organic Chemistry, M. McOmie, Editor, Plenum Press, N.Y., 1973 and U.S. Patent 4,617,149 which is incorporated herein by reference.

In selecting a particular protecting group, certain conditions must be observed. An $\alpha$-amino protecting group (1) must render the $\alpha$-amino function inert under the conditions employed in the coupling reaction, (2) must be readily removable after the coupling reaction under conditions that will not remove side chain protecting groups and will not alter the structure of the peptide fragment, and (3) must eliminate the possibility of racemization upon activation immediately prior to coupling. A side chain protecting group (1) must render the side chain functional group inert under the conditions employed in the coupling reaction, (2) must be stable under the conditions employed in removing the $\alpha$-amino protecting group, and (3) must be readily removable upon completion of the desired amino acid sequence under reaction conditions that will not alter the structure of the peptide chain.

It will be apparent to those skilled in the art that the protecting groups known to be useful for peptide synthesis will vary in reactivity to the agents employed for their removal. For example, certain protecting groups, such as triphenyl methyl and 2-(p-biphenylyl)isopropyloxycarbonyl are very labile and can be cleaved under mild acid conditions. Other protecting groups, such as t-butyloxycarbonyl, t-amyloxycarbonyl, adamantyloxycarbonyl, and p-methoxybenzyloxycarbonyl, are less labile and require moderately strong acids, such as trifluoroacetic, hydrochloric, or boron trifluoride in acetic acid, for their removal. Still other protecting groups, such as benzyloxycarbonyl, halobenzyloxycarbonyl, p-nitrobenzyloxycarbonyl, cycloalkyloxycarbonyl, and isopropyloxycarbonyl, are even less labile and require stronger acids, such as hydrogen fluoride, hydrogen bromide, or boron trifluoroacetate in trifluoroacetic acid, for their removal.

Upon completion of the desired peptide sequence, the protected peptide must be cleaved from the resin support, and all protecting groups must be removed. The cleavage reaction and removal of the protecting groups may be accomplished simultaneously or stepwise. When the resin support is a chloromethylated polystyrene resin, the bond anchoring the peptide to the resin is an ester linkage formed between the free carboxyl group of the C-terminal moiety and one of the many chloromethyl groups present on the resin matrix. It will be recognized that the anchoring bond can be cleaved by reagents which are known to be capable of breaking an ester linkage and of penetrating the resin matrix. One especially convenient method is by treatment with liquid anhydrous hydrogen fluoride. This reagent not only will cleave the peptide from the resin but will also remove all protecting groups. Hence, use of this reagent will directly afford the fully deprotected peptide. When it is desired to cleave the peptide without removing protecting groups, the protected peptide-resin can undergo methanolysis to give the protected peptide in which the C-terminal carboxyl group is methylated. The methyl ester can then be hydrolyzed under mild, alkaline conditions to give the free C-terminal carboxyl. The protecting groups on the peptide chain then can be removed by treatment with a strong acid, such as liquid hydrogen fluoride. A particularly useful technique for methanolysis is that of G. Moore et al., Peptides, Proc. 5th Amer. Pept. Symp., M. Goodman and J. Meienhofer, Eds., John Wiley, N.Y., 1977, pp. 518-521, in which the protected peptide-resin is treated with methanol and potassium cyanide in the presence of crown ether.

Another method for cleaving the protected peptide from the resin is by ammonolysis or by treatment with hydrazine. If desired, the resulting C-terminal amide or hydrazide can be hydrolyzed to the free C-terminal carboxyl, and the protecting groups can be removed conventionally.

It will also be recognized that the protecting group present on the N-terminal a-amino group may be removed preferentially either before or simultaneous with the cleavage of the protected peptide from the resin support.

The A and B chains of the insulin analogs of the present invention can also be prepared via recombinant DNA methodology. In their preparation, a nucleotide sequence coding for the desired peptide of the A or B chain is prepared using now-routine techniques for such synthesis. These methods generally involve preparation of oligonucleotides coding both for fragments of the desired coding sequence and for the complementary sequence thereof. The oligonucleotides are designed to provide overlap of one fragment of the coding sequence with two fragments of the complementary sequence and vice versa. The oligonucleotides are paired and joined, ultimately producing the desired gene sequence.

The sequence is inserted into a cloning vector at a location which permits the peptide product for which it codes to be expressed. The construction of plasmids capable of expressing proinsulin and proinsulin analogs is described in Chance et. al., European Patent Publication No. 0 383 472, published August 22, 1990, the entire teaching of which is herein incorporated by reference.

The A and B chains of the insulin analogs of the present invention may also be prepared via a proinsulin-like precursor molecule using recombinant DNA techniques. See Frank et al., Peptides: Synthesis-Structure -Function, Proc. Seventh Am. Pept. Symp., Eds. D. Rich and E. Gross (1981) which is incorporated herein by reference.

The step of combining the individual A and B chains, however produced, may be achieved by the method of Chance et al., Peptides: Synthesis, Structure and Function:Proc. of Seventh American Peptide Symposium (1981) which is incorporated herein by reference.

The following examples are provided as a means of illustrating the present invention and are not to be construed as a limitation thereon.

Example 1

Ala (B26) Human Insulin

The titled insulin analog was prepared by enzymatic semisynthesis (reverse proteolysis) using despentapeptide (B26-30) human insulin and synthetic pentapeptide Ala-Thr-Pro-Lys-Thr. The pentapeptide was prepared by solid-phase peptide synthesis whereas the despentapeptide human insulin was derived from biosynthetic human insulin (Eli Lilly and Company, Indianapolis) by a pepsin hydrolysis reaction that removed sequence 26-30 in substantial accordance with the teaching of Gattner, H.G., (1975) Hoppe-Seyler's Z. Physiol. Chem., 356: 1397-1404.

The peptide was synthesized on either an ABI synthesizer 430A or an ACT I Model 200 synthesizer. t-Boc-Thr(Bzl)OCH$_2$-PAM resin as well as protected t-Boc-amino acids and most pre-packaged reagents for peptide synthesis were purchased from Applied BioSystems, Inc. The pentapeptides were cleaved from the resin by regular HF cleavage. The cleavage was performed in 90% HF and 10% m-cresol at O°C for 1 hour and the HF was evaporated under vacuum. The peptide was precipitated immediately with cooled, anhydrous ether and washed again with cooled, anhydrous ether several times then finally dissolved in 10% acetic acid and lyophilized.

For the preparation of the titled insulin analog, 400 mg of des-pentapeptide human insulin and 730 mg of synthetic pentapeptide Ala-Thr-Pro-Lys-Thr were combined in 36 ml of a solution containing 85% 1,4-butanediol and 15% 1$\underline{M}$ Tris and the pH was adjusted to pH 7.0-7.1 using concentrated HCl. Forty (40) mg chymotrypsin was dissoved in 400 $\mu$l of 0.01 $\underline{N}$ HCl prior to being added to the protein solution. The coupling reaction was maintained at 25°C for 4.5 hours and monitored by reverse phase HPLC then terminated by adding 10 volumes of chilled acetone. The precipitate was collected by centrifugation, washed with ether, dissolved in 25 ml of 10% acetic acid containing enough guanidine HCl for solubilization, then applied to a Sephadex G50 (Superfine, 5 X 200 cm) column and eluted with molar acetic acid at 4°C. The desired fractions were pooled and subjected to a reverse phase HPLC column, Zorbax C8(2.12 X 25 cm), eluted with a linear gradient of 45-70% B buffer in 760 minutes at 2.5 ml/minute. The elution buffers were buffer A containing 0.1 M $NaH_2PO_4$, pH 2.1, and buffer B containing 50% buffer A and 50% $CH_3CN$. Further purification was conducted on a Vydac C18 column (2.12 x 25 cm) and the elution consisited of a linear gradient of 45-70% B buffer, 2.5 ml/minute. The mobile phase consists of solutions of 0.1% TFA as buffer A and 0.1% TFA/50% $CH_3CN$ as buffer B. Finally, 38 mg of lyophilized protein was obtained and and subjected to various analyses. Results of Fast Atom Bombardment Mass Spectrometry (FAB/MS) gave a molecular weight of 5715.6 (expected: 5715.4). The amino acid composition was as follows based on aspartic acid as molar unity (theoretical amino acid ratios in parenthesis), Asp, 3.00 (3); Thr, 2.89 (3); Ser, 2.75 (3); Glu, 7.24 (7); Pro, 1.06 (1); gly, 4.00 (4); Ala, 2.00 (2); Cys, 5.24 (6); Val, 3.36 (4); Ile, 1.41 (2); Leu, 6.12 (6); Tyr, 2.83 (3); Phe, 2.87 (3); His, 2.22 (2); Lys, 0.99 (1); Arg, 1.00 (1).

Other insulin analogs of the present invention were prepared in substantially the same manner as set forth above. Using the pentapeptide Gly-Thr-Pro-Lys-Thr as a starting material, the insulin analog Gly(B26)HI was prepared. Using the pentapeptide Val-Thr-Pro-Lys-Thr as a starting material, the analog Val(B26) HI was prepared. Using the pentapeptide Leu-Thr-Pro-Lys-Thr as a starting material, the analog Leu(B26) HI was prepared. Using the pentapeptide Ile-Thr-Pro-Lys-Thr as a starting material, the analog Ile(B26) HI was prepared. Using the pentapeptide Pro-Thr-Pro-Lys-Thr as a starting material, the analog Pro(B26) HI was prepared. Tests of each of these analogs demonstrated that these compounds are useful as fast acting insulin analogs.

## Example 2

### Size-Exclusion HPLC

Various concentrations of Ala(B26)HI were chromatographed with a DuPont Zorbax GF-250(0.94 x 25 cm) column eluted with mobile phase containing 0.2 M NaCl, 20mM $Na_2HPO_4$ and 0.001% $NaN_3$, pH 7.5. The column was run at 1 ml/minute and at 24°C. The distribution coefficient (Kd) is associated with the average radii of the molecule. In the equation Kd=(Ve-Vo)/(Vi-Vo), Ve is the elution volume of the sample, Vo is excluded volume determined by the elution of Blue Dextran, and Vi is the included volume determined by the elution of DL-dithiothreitol. The distribution coefficient (Kd) of insulin decreases significantly with increasing insulin concentration which indicates that insulin tends to aggregate at high concentrations. The replacement of the tyrosine residue at B26 of insulin with an alanine residue created an insulin analog which seemed to eliminate the aggregation under certain conditions and lead to concentration-independent Kd.

## Example 3

### Equilibrium Denaturation

Equilibrium denaturation experiments were performed according to the teaching of Brems $\underline{et}$ $\underline{al}$, (1990) $\underline{Biochemistry}$ 29:9289-9293, the entire teaching of which is incorporated herein by reference. The equilibrium denaturation was determined by far-UV circular dichroism with increasing guanidine concentrations. The denaturation transition of Ala(B26)HI began at 3.3 $\underline{M}$ GdnHCl. The free energy of unfolding ($\Delta$G) was 4.5 kcal/mol for Ala(B26)HI which is the same as for BHI. The GdnHCl concentration of the midpoint ([denatured]/[native]=1) was 5.3 $\underline{M}$ for Ala(B26)HI compared to 5.0 $\underline{M}$ for human insulin previously determined by Brems, et. al., 1991, $\underline{J.\ Biol.\ Chem.}$, $\underline{266}$:1611-1615.

## Example 4

### Equilibrium Ultracentrifugation

The aggregation behavior of the insulin was determined by sedimentation equilibrium ultra-centrifugation. The association state of each analog was measured using the techniques described in Pekar, A.H. and Frank, B.H. (1972) Biochemistry 11:4013-4016, the entire teaching of which is herein incorporated by reference. The proteins were dissolved in a 50mM sodium chloride-50mM sodium phosphate buffer and the pH was adjusted to 7.2. The protein concentrations were determined using an Aviv 14 DS spectro-photometer (Lakewood, N.J.). On a mg/ml basis, the extinction coefficient of BHI was 1.05 while the extinction coefficient for Ala(B26)HI was 0.800. The monomer molecular weight of BHI was 5808 for BHI and 5716 for Ala(B26)HI. Zinc was added to portions of the solutions using a stock solution of $ZnCl_2$ whose concentration had been determined by atomic spectroscopy. The ratio of moles of zinc to moles of protein was always 0.5. Sedimentation equilibrium experiments were done at 22°C in a Spinco Model E analytical ultracentrifuge with a 6-hole titanium rotor and photoelectric scanning absorption optical system. Overspeeding techniques were used so that equilibrium was reached before the equilibrium scans were begun at 25 hours. Standard 12mm centerpieces were used for the more diluted solutions and custom made 3mm charcoal-filled epon centerpieces were used for concentrated solutions. Sedimentation was assumed to be ideal and the weight average molecular weights at the various radii were calculated from the expression:

$$M_w = RT/(1 - vp)w^2 \cdot 1/r \cdot 1/c \cdot dC/dr$$

where R is the gas constant, T is the absolute temperature, w is the speed, r is the radius, C is the concentration and p is the solvent density. Ala (B26)HI was assumed to have the same partial specific volume as BHI, namely 0.73 ml/g. The weight average molecular weight was divided by the monomer molecular weight and compared to total concentrations of protein. Ala (B26)HI demonstrated less of a tendency to self-associate than BHI. For example, at 3.5 mg/ml (or 100 units/ml), insulin has a weight average molecular weight that is about 6.5 times the monomer molecular weight. Ala(B26)HI demonstrated a weight average molecular weight of about 3.2 times the monomer molecular weight. In the presence of 0.5 mole of zinc per mole of protein, insulin is primarily a hexamer by 0.5 mg/ml. In contrast, Ala(B26)HI is much less associated. As protein concentrations increase, Ala(B26)HI associates to species above hexamer in molecular weight.

Example 5

Receptor Binding

The physiological effects of the insulin analogs of the present invention were shown in the following in vitro insulin receptor binding assay. The preparation of human placental membrane and the binding assay were carried out with a modified procedure described by Gruppuso et. al. (1988) J. Clin. Endocrinal. Metab. 67:194-197, the entire teaching of which is herein incorporated by reference. The procedure employed incubating 30 to 50 μg of human placental membrane protein with approximately 10 fmol of $^{125}$I-insulin and various concentrations of unlabeled insulin or analogs in a final volume of 500 μl if 100 mM HEPES, pH 7.8, 120 mM NaCl, 5 mM KCl, 1.2 mM $MgSO_4$, 8 mM glucose and 0.25% BSA for 18 hours at 4°C. Membranes were collected on glass fiber filters pre-treated with 0.1% polyethyleneimine by using a cell harvester (Skatron, Lier, Norway). Binding data were analyzed by fitting displacement curves to a four parameter model employing Prefit/Allfit for the determination of $EC_{50}$ (the dose required to replace half of maximal binding) values. Data from four experiments demonstrated that human insulin displayed an $EC_{50}$ of 0.34 +/- 0.05 nM while Ala(B26)HI displayed an $EC_{50}$ of 0.39 +/- 0.02 nM, which translates to a potency 89% of insulin.

Example 6

Animal Studies

The physiological effects of the insulin analogs of the present invention were shown in the following in vivo assay system.

Normal male Sprague Dawley rats from the Charles River Laboratories (Portage, MI) were used as test animals. They were obtained at a weight range of 160-180 gms and maintained for one week in animal rooms at 75°F with a controlled light cycle (lights on 7:00 a.m. - 7:00 p.m., lights off 7:00 p.m. - 7:00 a.m.) The animals were fed Purina rat chow 5001 ad libitum. Rats used for each assay were fasted for 16 hours before being used. They weighed about 200 gms when first used. On reaching a fasted weight of about 275 gm (over a period of three weeks), the animals were no longer used. Five contol animals and five experimental animals were used for the test. The proteins were dissolved in 0.05 N HCl (pH 1.6) to provide a stock solution of 100 μgm per ml. From this, a number of dilutions were made in normal saline which was injected subcutaneously into the rats. A 100 μl sample of blood was taken from the tail vein of each rat at zero time and 30 minutes, 1 hour, 2 hours, 3 hours and 4 hours after administration. Glucose was determined colorimetrically by a glucose oxidase method (Sigma Chemical Co.). The percent change in blood glucose from the zero time value was calculated for each rat and the final results were expressed as the mean percent change $\pm$ SEM in the experimental group corrected for the mean change in the control group for that day.

A dose-response curve was drawn from tests with different concentrations of the compound tested expressing the percentage of glucose change at the glucose nadir for each of the doses. From this curve an $ED_{50}$ value was determined as that subcutaneous dose ($\mu$g/kg) of the protein which gave half the maximal hypoglycemic response. The $ED_{50}$ of human insulin was 7.8 +/- 0.1 (n=3) whereas the $ED_{50}$ for Ala(B26)HI was 8.9 +/- 0.7 (n=2), which translates to a potency 88% of insulin.

Studies performed in a pig model also demonstrated that the Ala(B26)HI analog is useful as a rapid acting insulin. In comparison to human insulin, nearly two times the amount of Ala(B26)HI is absorbed within 40 to 60 minutes from the subcutaneous injection.

As noted previously, the insulin analogs of the present invention have a reduced propensity to dimerize or otherwise self-associate to higher molecular weight forms. Thus, upon administration of one or more of said analogs, a rapid onset of activity is achieved. The insulin analogs of the present invention are effective in treating hyperglycemia by administering to a patient in need thereof an effective amount of an insulin analog containing an alanine residue at position 26 of the B chain. As used herein the term "effective amount" refers to that amount of one or more insulin analogs of the present invention needed to lower or maintain blood sugar levels either therapeutically or prophylactically. This amount typically may range from about 10 units up to about 60 units or more per day (or about 0.3 to about 2 mg assuming approximately 29 units per mg). However, it is to be understood that the amount of the insulin analog(s) actually administered will be determined by a physician in light of the relevant circumstances including the condition being treated (i.e., the cause of the hyperglycemia) the particular analog to be administered, the chosen parenteral route of administration, the age, weight and response of the individual patient and the severity of the patient's symptoms. Therefore, the above dosage ranges are not intended to limit the scope of the invention in any manner.

The insulin analogs of the invention are administered to a patient in need thereof (i.e., a patient suffering from hyperglycemia) by means of pharmaceutical compositions containing an effective amount of at least one insulin analog of containing an alanine residue at position 26 of the B chain in combination with one or more pharmaceutically acceptable excipients or carriers. For these purposes, the pharmaceutical compositions may typically be formulated so as to contain about 100 units per ml or similar concentrations containing an effective amount of the insulin analog(s). These compositions are typically, though not necessarily, parenteral in nature and may be prepared by any of a variety of techniques using conventional excipients or carriers for parenteral products which are well known in the art. See, for example, Remington's Pharmaceutical Sciences, 17th Edition, Mack Publishing Company, Easton, PA, USA (1985) which is incorporated herein by reference. For example, dosage forms for parenteral administration may be prepared by suspending or dissolving the desired amount of at least one insulin analog containing an alanine residue at position 26 of the B chain in a nontoxic liquid vehicle suitable for injection such as an aqueous medium and sterilizing the suspension or solution. An accompanying vial or vehicle can be provided for purposes of mixing prior to administration. Pharmaceutical compositions adapted for parenteral administration employ diluents, excipients and carriers such as water and water-miscible organic solvents such as glycerin, sesame oil, groundnut oil, aqueous propylene glycol, N,N'-dimethylformamide and the like. Examples of such pharmaceutical compositions include sterile, isotonic, aqueous saline solutions of the insulin analog containing an alanine residue at position 26 of the B chain which can be buffered with a pharmaceutically acceptable buffer and which are pyrogen free. Additionally, the parenteral pharmaceutical formulation may contain preservatives such as phenol or meta-cresol. Agents to adjust pH of the final product such as sodium hydroxide or hydrochloric acid may also be used.

The insulin analogs of the present invention may also be formulated into pharmaceutical compositions suitable for intranasal administration. Such compositions are disclosed in detail in European Patent Application 0200383 A3 which is incorporated herein by reference. Briefly, such compositions are formulated with one or more pharmaceutically acceptable diluents, a pharmaceutically acceptable amount of an alkali metal salt, the ammonium salt, or the free acid of a substantially zinc-free insulin, and optionally, an absorption enhancing amount of at least one absorption enhancing agent selected from the group consisting of (1) oleic acid or an ester or salt thereof, (2) a liquid form sorbitan fatty acid ester, (3) a liquid form polyoxyethylene derivative of a sorbitan fatty acid ester, and (4) a liquid form hydroxypolyoxyethylenepolyoxypropylene-polyoxyethylene copolymer.

**Claims**

1.  An insulin analog of the formula SEQ ID NO:1 properly cross-linked to SEQ ID NO:2, or a pharmaceutically acceptable salt thereof, wherein Xaa at position 21 of SEQ ID NO:1 is selected from the group consisting of Asn, Asp, Glu, Gln, Ala, Gly or Ser, Xaa at position 1 of SEQ ID NO: 2 is selected from the group consisting of Asp or Phe, Xaa at position 3 of SEQ ID NO:2 is selected from the group consisting of Asp or Asn, and Xaa at position 26 of SEQ ID NO:2 is selected from the group consisting of Ala, Gly, Val, Leu, Ile and Pro.

2.  The insulin analog of Claim 1 wherein Xaa at position 21 of SEQ ID NO:1 is Asn.

3.  The insulin analog of Claim 2 wherein Xaa at position 1 of SEQ ID NO:2 is Phe.

4.  The insulin analog of Claim 3 wherein Xaa at position 3 of SEQ ID NO:2 is Asn.

5. The insulin analog of Claim 4 wherein Xaa at position 26 of SEQ ID NO:2 is Ala.

6. A pharmaceutical formulation comprising, in a pharmaceutically acceptable diluent, an insulin analog of the formula SEQ ID NO:1 properly cross-linked to SEQ ID NO:2 or a pharmaceutically acceptable salt thereof, wherein Xaa at position 21 of SEQ ID NO:1 is selected from the group consisting of Asn, Asp, Glu, Gln, Ala, Gly or Ser, Xaa at position 1 of SEQ ID NO: 2 is selected from the group consisting of Asp or Phe, Xaa at position 3 of SEQ ID NO:2 is selected from the group consisting of Asp or Asn, and Xaa at position 26 of SEQ ID NO:2 is selected from the group consisting of Ala, Gly, Val, Leu, Ile and Pro.

7. The pharmaceutical formulation of Claim 6 wherein Xaa at position 21 of SEQ ID NO:1 is Asn, Xaa at position 1 of SEQ ID NO:2 is Phe, Xaa at position 3 of SEQ ID NO:2 is Asn, and Xaa at position 26 of SEQ ID NO:2 is selected from the group consisting of Ala, Gly, Val, Leu, Ile and Pro.

8. The pharmaceutical formulation of Claim 7 wherein Xaa at position 26 of SEQ ID NO:2 is Ala.

9. A process for preparing an insulin analog as claimed in any on of claims 1 to 5, which comprises crosslinking a compound of SEQ ID NO:1 with a compound of SEQ ID NO:2.

10. An insulin analog whenever prepared by a process according to Claim 9.

SEQUENCE LISTING

(1)  GENERAL INFORMATION:

    (i)  APPLICANT:  ELI LILLY AND COMPANY
        (B)  STREET:    Lilly Corporate Center
        (C)  CITY:      Indianapolis
        (D)  STATE:     Indiana
        (E)  COUNTRY:   United States of America
        (F)  ZIP:       46285

    (ii) TITLE OF INVENTION:  Insulin Analogs

    (iii)NUMBER OF SEQUENCES:  2

    (iv) CORRESPONDENCE ADDRESS:
        (A)  ADDRESSEE:  C. M. Hudson
        (B)  STREET:  Erl Wood Manor
        (C)  CITY:  Windlesham
        (D)  STATE:  Surrey
        (E)  COUNTRY:  United Kingdom
        (F)  ZIP:  GU20 6PH

    (v)  COMPUTER READABLE FORM:
        (A)  MEDIUM TYPE:  Floppy disk
        (B)  COMPUTER:  Macintosh
        (C)  OPERATING SYSTEM:  Macintosh 7.0
        (D)  SOFTWARE:  Microsoft Word 5.1

(2)   INFORMATION FOR SEQ. NO. 1:

(i)   SEQUENCE CHARACTERISTICS:
      (A)   LENGTH:   21 amino acids
      (B)   TYPE:   amino acid
      (C)   STRANDEDNESS:
      (D)   TOPOLOGY:   linear

(ii)  MOLECULE TYPE:   polypeptide

(ix)  FEATURE:
      (A)   NAME/KEY:   Variable Site
      (B)   LOCATION:   21
      (C)   IDENTIFICATION METHOD:
      (D)   OTHER INFORMATION:   'This amino acid is
either Asn, Asp, Glu, Gln, Ala, Gly or Ser.'

(xi)  SEQUENCE DESCRIPTION:   SEQ. ID NO. 1:
Gly Ile Val Glu Gln Cys Cys Thr Ser Ile Cys Ser Leu Tyr Gln
 1               5                   10                  15

Leu Glu Asn Tyr Cys Xaa
              20

(3)   INFORMATION FOR SEQ NO. 2:

(i)   SEQUENCE CHARACTERISTICS:
      (A)   LENGTH:   30 amino acids
      (B)   TYPE:   amino acid
      (D)   TOPOLOGY:   linear

(ii)  MOLECULE TYPE: polypeptide

```
(ix)  FEATURE:
      (A)   NAME/KEY: Variable Site
      (B)   LOCATION:  1
      (C)   IDENTIFICATION METHOD:
      (D)   OTHER INFORMATION: 'This amino acid is
either Phe or Asp.'


(ix)  FEATURE:
      (A)   NAME/KEY: Variable Site
      (B)   LOCATION:  3
      (C)   IDENTIFICATION METHOD:
      (D)   OTHER INFORMATION: 'This amino acid is either
Asn or Asp.'


(ix)  FEATURE:
      (A)   NAME/KEY: Variable Site
      (B)   LOCATION:  26
      (C)   IDENTIFICATION METHOD:
      (D)   OTHER INFORMATION: 'This amino acid is either
Ala, Gly, Val, Leu, Ile or Pro.'


(xi) SEQUENCE DESCRIPTION:  SEQ ID NO. 2:
Xaa Val Xaa Gln His Leu Cys Gly Ser His Leu Val Glu Ala Leu
 1               5                   10                  15


Tyr Leu Val Cys Gly Glu Arg Gly Phe Phe Xaa Thr Pro Lys Thr
                20                  25                  30
```